# EUROPEAN PATENT SPECIFICATION

(11) **EP 0 904 097 B1**
(45) Date of publication and mention of the grant of the patent: **28.05.2003**
(21) Application number: 97917089.1
(22) Date of filing: 27.03.1997
(51) Int. Cl.: A61K 38/11, A61K 31/55, A61P 9/04

(54) **COMBINATIONS OF VASOPRESSIN AND ADRENERGIC AGENTS FOR THE TREATMENT OF CARDIAC ARREST**
KOMBINATIONEN VON VASOPRESSIN UND ADRENERGISCHEN WIRKSTOFFEN ZUR BEHANDLUNG DES PLÖTZLICHEN HERZTODS
ASSOCIATIONS DE VASOPRESSINE ET D'AGENTS ADRENERGIQUES POUR LE TRAITEMENT DES ARRETS CARDIAQUES

(30) Priority: 29.03.1996 US 625733
(43) Date of publication of application: 31.03.1999
(73) Proprietor: Lurie, Keith G., Minneapolis, MN 55409 (US); Lindner, Karl, 89075 Ulm (DE)
(72) Inventor: Lurie, Keith G., Minneapolis, MN 55409 (US); Lindner, Karl, 89075 Ulm (DE)
(74) Representative: Hay, Martin Alexander
(86) International application number: PCT/US97/05056
(87) International publication number: WO 97/036609

(56) References cited:
- LURIE K G ET AL: "Synergistic effects of vasopressin plus epinephrine during cardiopulmonary resuscitation" CIRCULATION, 94 (8 SUPPL.). 1996. I356., XP002037725
- WILSON M F ET AL: "MECHANISMS OF IMPAIRED CARDIAC FUNCTION BY VASOPRESSIN" ANN SURG, 191 (4). 1980. 494-500., XP002037726

## Description

### Background of the Invention

In the United States each year, more than 350,000 people die from cardiac arrest prior to arriving in the hospital. Even when patients are resuscitated initially, more than half die in the hospital within the first 24 hours. Eisenberg et al., NEJM, 306, 1340 (1982). Despite more than three decades of the practice of manual external chest compression or standard CPR, together with epinephrine administration, less than 5% of patients who suffer a cardiac arrest survive. Niemann, NEJM, 327, 1075 (1992). Though time to initiation of standard CPR is a critical factor in determining outcome, the inherent limitations of manual external chest compression are perhaps an even greater reason for the poor survival statistics. In light of the tremendous amount of time, money and energy involved in basic CPR performance and training, these statistics become even more disheartening. Although pharmacologic therapy, specifically intravenous epinephrine and antiarrhythmic therapies, have served to improve outcomes in some patients, the role of vasopressor agents during CPR remains controversial. Stiell et al., NEJM, 327, 1045 (1992).

Over the past 35 years since standard manual external chest compression was described, there have been a number of advances in the mechanical means available to improve overall CPR efficacy. Based on the assumption that increases in intrathoracic pressures will increase cardiac output during ventricular fibrillation, techniques such as the circumferential vest and active compression and decompression (ACD CPR) have been developed. Lurie, Resuscitation, 28, 115 (1994). In an effort to enhance filling of the coronary arteries and enhance venous return during the diastolic or decompression phase of CPR, techniques such as interposed abdominal counterpulsion CPR and use of a rapidly inflating and deflating intra-aortic balloon pump, as well as ACD CPR have been used. Sack et al., JAMA, 267, 379 (1992).

In addition to the research conducted on mechanical means to improve the efficacy of CPR, there has been a renewed interest in developing pharmacological therapies to improve the vital organ blood flow and overall survival of patients who have suffered a cardiac arrest. Such therapies typically include the intravenous administration of epinephrine during the performance of CPR. Epinephrine is an arterial constrictor, and its use is intended to enhance patient blood pressure during the resuscitation process. Even with epinephrine, however, survival after cardiac arrest is poor. Furthermore, recent studies have demonstrated no added benefit from doses of epinephrine higher than the traditional dose. Stiell et al., cited supra. Recent studies show no benefit of high or low dose epinephrine with placebo. (Woodhouse et al., Resuscitation, 30, 243 (1995).

ANN SURG , 191 (4), 1980, pages 494 to 500, discloses that vasopressin impairs cardiac function and that the addition of epinephrine to vasopressin restored the mechanical performance of the heart to normal.

Applicant has described the concurrent administration of a venodilator (nitroglycerin) and an arterial constrictor (epinephrine) during CPR in published PCT application WO 94/11045, which discloses a method for resuscitating a patient from cardiac arrest, comprising actively inducing venous blood transport into the heart and arterial blood transport from the heart; ventilating the patient's lungs; administering to the patient concurrently with said inducing and ventilating steps an arterial constrictor sufficient to increase the patient's arterial blood pressure; and administering to the patient concurrently with said inducing and ventilating steps an amount of a venodilator sufficient to enhance arterial blood flow to the patient's brain and heart.

However, a continuing need exists for improved methods and pharmaceutical compositions for use during CPR, which result in enhanced long term survival among at least certain populations of cardiac arrest patients. Such methods and compositions would preferably enhance blood circulation and delivery of oxygenated blood to patient tissue, particularly heart and/or brain tissue, without significantly lessening patient blood pressure.

### Summary of the Invention

Surprisingly, it has been found that the use of vasopressin when combined with an adrenergic agent such as epinephrine or aminophylline, leads to enhanced vital organ blood flow during CPR when compared with conventiqnal epinephrine therapy. Additionally, Applicant has discovered that the use of vasopressin with a bradycardic agent such as zatebradine, or furthermore, that the use of one or more bradycardic agents in combination with one or more adrenergic agents, results in surprisingly enhanced efficacy of CPR. Thus, the present invention provides the use of vasopressin in conjunction with one or more adrenergic agents for the preparation of a medicament to treat cardiac arrest when administered to a patient undergoing CPR.

The present agents or medicaments are conveniently presented in kit form, i.e., as one packaged unit comprising the aforementioned combinations of active ingredients. Accordingly, the present invention also provides kits for the treatment of cardiac arrest, and, in particular, for the treatment of ventricular fibrillation, asystole, electromechanical dissociation (EMD), and the like. Specifically, the kits comprise packaging material containing the aforementioned agents packaged in unit dosage forms singly or in combination, and instruction means indicating that said agents are to be administered to a patient undergoing cardiopulmonary resuscitation.

Preferably, the bradycardic agent is zatebradine, tedisamil, falipamil, mixidine alinidine or a β-adrenergic receptor blocker such as propanolol, metoprolol or esmolol. More preferably, the bradycardic agent is zatebradine. If zatebradine is to be administered, or included in a kit, it is preferred that it be administered or present in one or more unit dosage forms, each comprising about 10 to about 120 mg zatebradine.

It is further preferred that the adrenergic agent is an adrenergic agonists such as epinephrine, dopamine, norepinephrine, isoproterenol, phenylephrine, dobutamine or methoxamine. Most preferably, the adrenergic agonist is epinephrine. The adrenergic agent can also be an indirect sympathomimetic agent which prevents the degradation of cAMP, i.e., a phosphodiesterase (PDE) inhibitor. A representative PDE inhibitor is aminophylline. Administration of more than one adrenergic agent, for example epinephrine and isoproterenol, plus vasopressin will also enhance vital organ blood flow during CPR. In this manner, adrenergic agents with relatively great α and/or β adrenoceptor selectivity can be combined and used in conjunction with vasopressin therapy.

The vasopressin, bradycardic agent and/or the adrenergic agent can be administered to a patient suffering from cardiac arrest and undergoing active inducement of venous blood transport into the heart and arterial blood transport from the heart, in amounts effective to increase arterial blood pressure, so as to enhance arterial blood flow to the brain and heart. More preferably, a unit dosage form of the vasopressin will comprise about 10 units to 120 units, a unit dosage form of the epinephrine will comprise about 0.25 mg to 3.0 mg, a unit dosage form of zatebradine will comprise about 5 mg to 150 mg, a unit dosage form of propanolol will comprise about 0.25 mg to about 3 mg and a unit dosage form of esmolol will comprise about 5 mg to about 100 mg.

Applicant has made the surprising discovery that the administration of an organic nitrate with vasodilatory properties, such as nitroglycerin, to a patient in cardiac arrest concurrently with vasopressin and/or an adrenergic agent or bradycardic agent attenuates some of the vasoconstrictor effects of the latter agents on the coronary arterial bed. The administration of an organic nitrate serves to dilate the coronary vessels and decrease preload without a significant effect on afterload when administered concurrently with the pharmaceutical composition of the present invention. Thus, the use may optionally be performed in further conjunction with, and the kit may optionally comprise, an amount of an organic nitrate effective to increase myocardial blood flow. Preferably, the organic nitrate will be nitroglycerin and will be administered (or present in a unit dosage form in the kit) at a dose range of about 10 µg to 300 µg.

In addition to the vasopressin and the adrenergic agent, the use may be performed in further conjunction with, and the kit may include unit dosage forms of, other active substances which are intended to enhance the therapeutic effectiveness of the active agents. For example, the use may be performed in conjunction with, and the kit can also include, mannitol in an amount effective to reduce swelling of the brain, heart, and/or kidneys, due to mannitol's osmotic properties. Additionally, mannitol can also enhance patient blood pressure. Mannitol can be provided at a total dosage in the range from about 1 g to 100 g, preferably from about 5 g to 50 g.

A calcium channel blocker, such as diltiazem, verapamil, nifedipine, and the like, may also optionally be administered in conjunction with the other active ingredients or included in the kit of the present invention in an amount effective to inhibit calcium overload. Typically, a unit dosage form of diltiazem would comprise about 0.5 mg to 60 mg, preferably from about 0.5 mg to 20 mg. A unit dosage form of verapamil would comprise about 0.5 mg to 60 mg, preferably from about 0.5 mg to 5 mg. A unit dosage form of nifedipine would be present in a dosage from about 0.2 mg to 10 mg, preferably from about 0.5 mg to 5 mg.

### Brief Description of the Figures

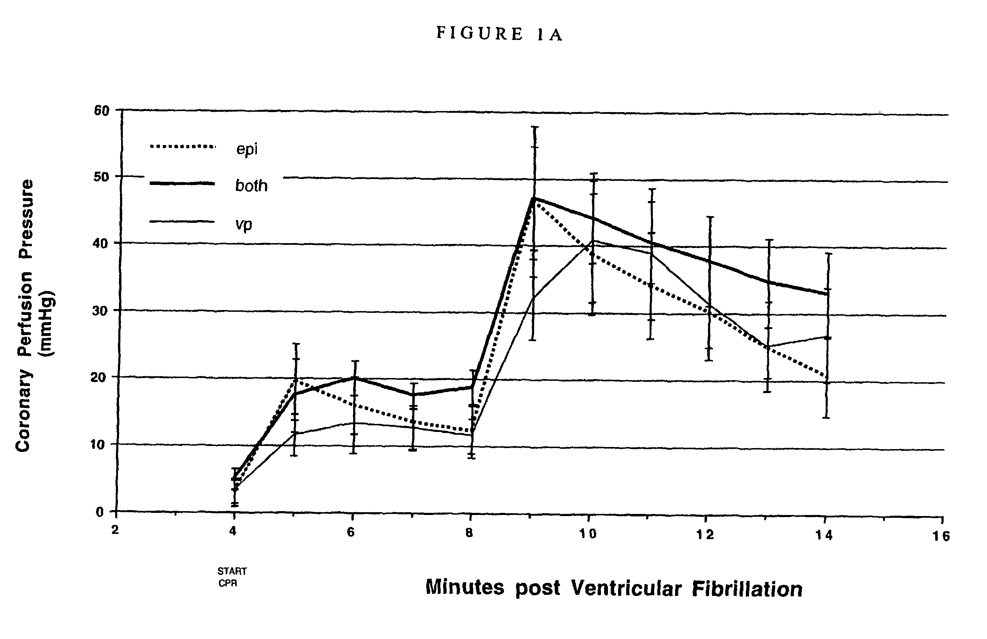

Figure 1a is a graphical depiction of the coronary perfusion pressures (CPP) (diastolic aortic minus right arterial pressures) of pigs (n=6 per group) resuscitated from ventricular fibrillation with epinephrine alone (EPI) 40 µg/kg, vasopressin alone (VP) 40 units, or a combination of epinephrine and vasopressin (EPI + VP) which illustrates that the combination of vasopressin + epinephrine leads to more rapid, higher, and more sustained coronary perfusion pressures (mean ± SEM) than either agent alone. After 14 minutes of CPR and 10 minutes after drug, the mean coronary perfusion pressure was 50% greater with vasopressin plus epinephrine then epinephrine alone. The benefit of the combined approach is similarly seen after 1 minute where the higher perfusion pressure are seen on the combined regimen versus vasopressin along.

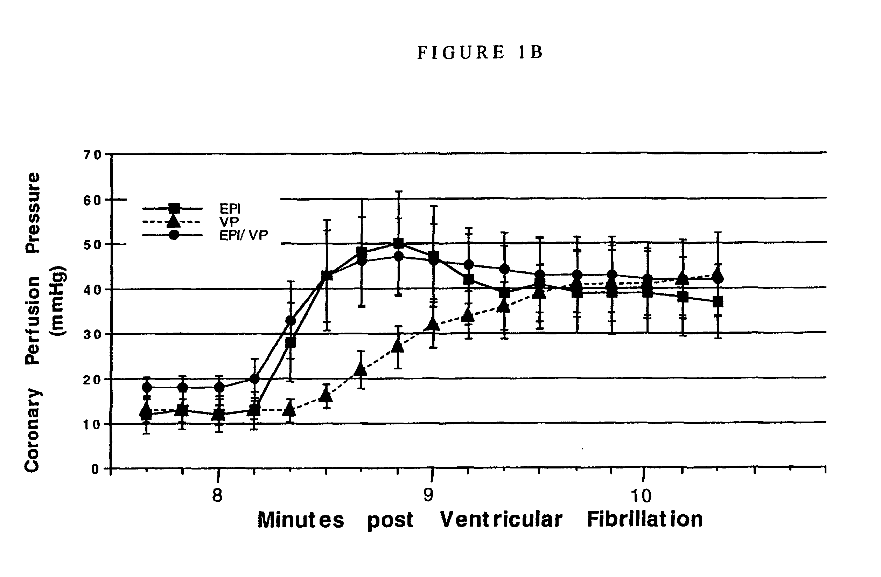

Figure 1b shows the slower onset of effects of vasopressin compared with epinephrine alone or in combination with vasopressin.

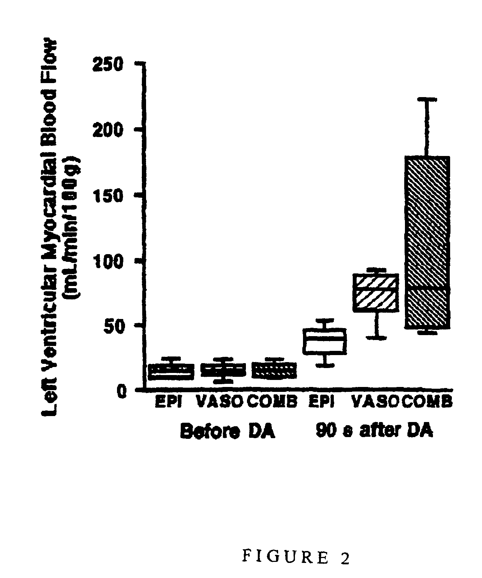

Figure 2 is a graphical depiction of the results of a series of experiments conducted with higher concentrations of vasopressin (0.4 units/kg) and epinephrine (0.2 mg/kg) in a porcine model of ventricular fibrillation using standard CPR which illustrates that median (box = upper and lower quartiles, bars are maximal/minimal valves) myocardial and cerebral blood flows were increased by nearly 40% with the combination of these agents when compared with each drug alone.

### Detailed Description of the Invention

Cardiac arrest generally refers to conditions resulting in the loss of effective heart function and the loss of effective blood circulation. Specific conditions treatable by the present invention include ventricular fibrillation, characterized by rapid contractions and twitching of the heart muscle; asystole, characterized by the substantial absence of contractions of the heart; and electromechanical dissociation (EMD), characterized by the persistence of electrical activity in the heart without associated mechanical contractions.

### A. Methods of Cardiopulmonary Resuscitation

The kit of the present invention provides packaging material comprising unit dosage forms of vasopressin and one or more adrenergic agents; and instruction means indicating that said agents are to be administered to a patient undergoing cardiopulmonary resuscitation. The aforementioned agents may be packaged in unit dosage forms singly or in combination. The active agents, when administered during the active inducement of blood transport and lung ventilation, act to, in the instance of vasopressin and'adrenergic agents, increase arterial blood pressure and enhance arterial blood circulation, and in the instance of bradycardic agents, act to decrease the heart rate and cardiac metabolism. The active inducement of blood transport includes both the transport of venous blood from the extremities and abdomen, into the thorax and heart, as well as the transport of blood from the heart into the lungs and arterial system. Both the induced blood transport and the lung ventilation are preferably achieved by certain advanced CPR methods, such as ACD CPR, as described in Cohen et al., JAMA, 267, 2916 (1992), and interposed abdominal compression (IAC), as described in Neimen, N. Engl. J. Med., 327, 1075 (1992). Induced blood transport and lung ventilation can be achieved, but to a lesser extent, with standard chest massage and CPR techniques. Optionally, additional measures will be taken to provide lung ventilation, such as use of an endotracheal tube, mouth-to-mouth resuscitation, or the like.

### B. Pharmacologic Therapy for Patients in Cardiac Arrest

While the uses of the present invention can by themselves enhance the efficacy of CPR so as to improve the chances for patient survival when compared to conventional CPR, the present invention is based on the discovery that the vital organ blood flow (with conventional CPR and in particular with the enhanced CPR techniques described above) can be further improved by administering the combination of vasopressin, a bradycardic agent and/or an adrenergic agent. It has also been discovered that the administration of organic nitrates with vasodilatory properties, such as nitroglycerin, in combination with the combinations of active agents of the present invention, can attenuate some of the intense vasoconstrictor effects of the exogenous vasopressors on the coronary arterial bed and thereby further improve myocardial blood flow.

### 1. Vasopressin

Vasopressin is usually called antidiuretic hormone (ADH) by physiologists and biochemists, because it decreases urine flow by increasing the resorption of water from the distal convoluted tubules and collecting ducts of the kidney. Not only does it promote water retention but under certain circumstances, it increases the excretion of sodium and chloride. The effect is a decrease in the osmolarity of the extracellular fluid. In addition to its antidiuretic effects, vasopressin also stimulates vascular smooth muscles.

When compared with "optimal" doses of epinephrine administered during CPR, vasopressin administration results in higher levels of myocardial perfusion, greater cerebral perfusion, and greater chances for resuscitation. Lindner et al., Anesthesia & Analgesia, 77, 427 (1993). Furthermore, the higher the level of vasopressin in humans in cardiac arrest, the greater the chances of survival. Lindner et al., Br. Heart J., 75, 145 (1996). In contrast, the higher the level of endogenous levels of catecholamines in patients in cardiac arrest, the worse the chances of survival. Vasopressin is effective as an arterial constrictor under conditions of severe acidosis and the duration of action of vasopressin appears to be 3-4 times longer than comparable doses of epinephrine. Lindner et al., Circulation, 91, 215 (1994). Vasopressin is commercially available from Park Davis, Morris Plains, New Jersey.

### 2. Adrenergic agents.

### a. Adrenergic agonists (direct-acting sympathomimetics)

Adrenergic agonists increase the heart rate, enhance atrioventricular conduction, and increase the strength of the heart beat (positive inotropic action). They also induce lipolysis and thus increase the concentration of plasma free fatty acids. These effects are achieved, in part, through the activation of the adenylcyclase system and the intermediation of 3', 5'-cyclic adenosine monophosphate (cyclic AMP). Adrenergic agonists suitable for use in the present invention include, but are not limited to, epinephrine, norepinephrine, dopamine, dobutamine, isoproterenol, phenylephrine, methoxamine, and the like. Preferably, the adrenergic agonist employed is epinephrine.

### (1) Epinephrine

Epinephrine is the predominant sympathomimetic in the adrenal medulla and stimulates both α and β adrenoceptors. β₁ as well as β₂ stimulation may be particularly important in the heart especially immediately after direct current shock. It is liberated in conditions of stress and vigorous exertion. At low doses or low intravenous infusion rates, it is possible to stimulate the heart and relax bronchioles and at the same time decrease the diastolic blood pressure. However, the vasoconstrictor effect is stronger than the vasodilator effect, so that at higher doses there is a net increase in vascular resistance and extreme hypertensive crisis can occur with overdoses. Epinephrine is the drug of choice in the management of allergic emergencies, such as anaphylaxis, edema, and the like. Epinephrine is also a resuscitant for patients in cardiac arrest, however, the concomitant vasoconstriction is thought to place an unwanted load on a compromised heart. Epinephrine is commercially available from a number of companies.

### b. Indirect Sympathomimetic Agents (Phosphodiesterase Inhibitors)

The phosphodiesterase inhibitors have been recognized as potent inotropic and vasodilating drugs. In acute congestive heart failure, they increase cardiac output, decrease left pulmonary capillary wedge pressure, and reduce total peripheral resistance with an improvement in loading conditions of the failing heart. Their potency in reversal of symptoms of acute congestive heart failure is quite similar to, or even better than, treatment with intravenous catecholamines and sodium nitroprusside. A preferred PDE inhibitor for use in the present method is aminophylline. See Merck Index (11th ed.) at entry 477. Other suitable PDE inhibitors are disclosed by T.A. Fischer et al., Drugs, 44, 928 (1992).

### 3. Bradycardic Agents

Bradycardic agents decrease heart rate and assist in the resuscitation process. Specifically, these agents help to decrease myocardial oxygen consumption (which is increased by adrenergic agents). β-adrenoceptor antagnoists funtion as bradycardic agents by blocking the effects of β-adrenergic agonists in the heart. Other bradycardic agents, such as zatebradine, work in part by blocking the potassium channels including the mixed sodium/potassium pacemaker current I_{f}. During a cardiac arrest, these agents are particularly helpful immediately after return of spontaneous circulation.

### a. Zatebradine

Zatebradine is a bradycardic agent that acts as a blocker of the mixed sodium/ potassium pacemaker current If and inhibits sinus node function. Kobinger et al., Eur. J. Pharmacol., 104, 9 (1984). Although zatebradine is a structural analog of verapamil, this drug does not possess classical calcium channel antagonist activity. Id. In isolated tissue preparations, this compound has been shown to produce marked negative chronotropic actions without any negative inotropic effects. Furthermore, in conscience dogs, zatebradine administration results in a decrease in heart rate with little change in arterial blood pressure. Id. It is this ability of zatebradine to selectively reduce heart rate without producing other hemodynamic alterations that make it highly useful in the perioperative setting. Riley et al., Anesthesiology, 67, 707 (1987).

### 4. Organic Nitrates

Organic nitrates act physiologically via nitric oxide. They are potent vasodilators which act more on the venous or capacitance beds than on the peripheral arterial beds. These agents also cause a marked vasodilation of the coronary arteries and arterioles, leading to an increase in endocardial blood flow. The organic nitrates are particularly effective under conditions of cardiac ischemia and heart failure.

### a. Nitroglycerin

Nitroglycerin is a coronary dilator and a general relaxant of smooth muscle. Its actions are directly on the smooth muscle. It preferentially dilates capacitance veins when compared with system resistance arteries. In the doses used for prophylaxis or relief of acute attacks of angina pectoris, it dilates the capacitance veins, which decreases venous return, and the coronary arteries, which decreases arterial impedance.

### C. Modes of Administration

The aforementioned drugs (or "active agents"), i.e., vasopressin, bradycardic agent(s), adrenergic agent(s) and organic nitrate(s), are preferably administered to the patient concurrently with or as soon as possible after the initiation of the enhanced CPR procedure, preferably being administered within from 0 to 60 minutes after such initiation, more preferably being administered from 0 to 10 minutes after such initiation. Both the initiation of CPR and the administration of the combination of active ingredients should be initiated as shortly as possible after the cardiac arrest, with drug administration preferably beginning within 10 minutes of arrest. Administration of the combination dose of drugs will preferably be repeated during procedures which last for more than 10 minutes, usually being repeated every 3 to 10 minutes.

The drugs can be administered by any technique which assures rapid absorption into patient circulation, preferably being administered parenterally, i.e., by injection or infusion, as intravenously, endotracheally, sublingually,intracardiac, or by other parenteral routes. Intravenous injections will usually be made to a peripheral or central vein in a conventional manner. Endotracheal administration may also be performed and is particularly suitable if an endotracheal tube has been introduced in order to enhance lung ventilation and intravenous access is not immediately available. Devices and methods suitable for endotracheal administration of drugs according to the present invention are described in U.S. Patent No. 4,693,243. In the case of endotracheal administration, the total dosages described above will generally be increased in order to offset the inefficiencies of such an administration route. The dosages will usually be increased from two-fold to three-fold.

The drugs will preferably be administered together in a single dosage or bolus, but could also be administered separately and/or sequentially to the patient. Furthermore, the total desired dosage of each of the drugs may be administered in two or more discrete boluses.

### 1. Dosage Forms

The aforementioned drugs can be formulated for administration to a human patient in cardiac arrest in one or more unit dosage forms comprising an effective amount of vasopressin, bradycardic agent, and/or an adrenergic agent, optionally further comprising an organic nitrate, mannitol and/or a calcium channel blocker, in combination with a pharmaceutically acceptable liquid carrier, such as distilled water, physiological salt solutions such as normal saline, buffers, and the like. Such dosage forms will typically include a pharmaceutically acceptable preservative, and may include other components commonly employed in solutions suitable for intravenous and/or endotracheal administration such as nontoxic surfactants.

Unit dosage forms comprising the active agents can be formulated so as to deliver single or multiple dosages of the drugs parenterally, as by injection or infusion. Such dosage forms include prefilled bottles, ampules, plastic bags or preloaded syringes. Methods for preparing such pharmaceutical compositions and unit dosage forms are well known in the art and described in more detail in various sources including, for example, *Remington's Pharmaceutical Science*, 15th Edition, Mack Publishing, Easton, Pennsylvania (1980).

Unit dosage forms suitable for injection or infusion can include sterile concentrated aqueous or aqueous-alcoholic solutions or dispersions which are adapted for extemporaneous dilution to yield sterile injectable or infusible solutions or dispersions. In all cases, the ultimate dosage form must be sterile, fluid and stable under the conditions of manufacture and storage. The liquid carrier or vehicle can be a solvent or liquid dispersion medium comprising, for example, a major portion of water in combination with ethanol, a polyol (for example, glycerol, propylene glycol, and liquid polyethylene glycols, and the like), vegetable oils, nontoxic glycerol esters, lipids (for example, dimyristoyl phosphatidyl choline) and suitable mixtures thereof. The proper fluidity can be maintained, for example, by the formation of liposomes, by the maintenance of the required particle size in the case of dispersion or by the use of nontoxic surfactants. The proper pH can be maintained with a variety of different buffers, for example, citrate or acetate. Sterile injectable or infusible solutions are prepared by incorporating the vasopressin, bradycardic agent, organic nitrate and/or an adrenergic agonist in the required amount in the appropriate solvent with various of the other ingredients enumerated above, and as required, followed by filter sterilization.

### 2. Dosages

The dosage of the vasopressin, bradycardic agent, adrenergic agent and organic nitrate to be utilized in the use or kit of the present invention can be varied widely, in accord with the size, age and condition of the patient. However, it is preferred that the vasopressin be administered in a dosage of from about 10 units to 120 units and that the adrenergic agent, if, for example, it is to be epinephrine, is administered in a dosage of from about 0.25 mg to 2.0 mg (i.e., total amount given to the patient at one time point during the procedure; administration may be repeated at successive time points as described below).

It is further preferred that the bradycardic agent, if, for example it is to be zatebradine, is administered in a dosage of about 5 mg to about 150 mg. If the bradycardic agent to be administered is to be a β-adrenergic receptor blocker, e.g., propanolol, it is preferred that it be administered in a dosage of about 0.25 mg to about 3 mg, or if the β-adrenergic receptor blocker is to be esmolol it is preferably administered in a dosage of about 5 mg to about 100 rng. If an organic nitrate is to be administered, e.g. nitroglycerin, it is preferred that it is administered in a dosage from about 10 µg to about 300 µg. Most preferably, the vasopressin is administered in a dosage of from about 10 units to about 50 units and the epinephrine is administered in a dosage of from about 0.75 mg to about 1.25 mg. The above dosages are the preferred ranges for adults and would be reduced somewhat for administration to children and infants.

The invention will be further described by reference to the following detailed examples.

### Example 1.

Applicants believe that concurrent administration of an adrenergic agonist together with vasopressin may help restore sinus nodal function and/or enhance myocardial contractility after cardioversion. Moreover, since vasopressin is more effective in acidosis and has a longer duration of action, Applicants believe that it may be better for helping to restore myocardial perfusion before cardioversion. This hypothesis was tested by comparing the effects of epinephrine (EPI) (40 ug/kg) versus vasopressin (VP) (0.3 units/kg) alone or in combination.

### 1. Materials and Methods

### a. Preparation of pigs.

Healthy female domestic farm pigs (28-33 kgs) were fasted overnight and anesthetized with pentobarbital (20 mg/kg IV bolus followed by 2.5 mg/kg/hr IV infusion) via an ear vein. Once anesthetized, pigs were placed in the dorsal recumbent position and intubated using standard endotracheal intubation technique. They were ventilated during the preparatory phase of the experiment and after return of spontaneous circulation at the end of the experiment with a mechanical respirator (model 607, Harvard Apparatus Co., Inc., Dover, MA). The tidal volume was set at 450 cc and delivered between 11 and 15 breaths per minute with supplemental oxygen at 2 liters/minute. Normal saline solution will be administered intravenously through the preparative and study periods using an infusion pump (Flo-Gard 6201, Baxter Healthcare, Deerfield, IL).

The preparatory phase, which includes cannulation of both femoral arteries and the right jugular vein, as well as calibration of all instruments, takes approximately 2 hours. Once venous access was obtained, animals received normal saline solution at approximately 300-400 ml/hr to maintain diastolic right atrial pressures of 3-5 mm Hg. Arterial blood gases were analyzed every 30 minutes to insure adequate acid base status and oxygenation. Left ventricular and ascending aortic arch blood pressures were monitored using a single high fidelity micronanometer catheter (Millar, Houston, TX). This aorto-left ventricular catheter has a lumen for injecting radiolabeled microspheres and it was positioned, under fluoroscopic guidance, 15 minutes prior to initiation of VF. Right atrial pressures were monitored using a micromanometer catheter (Millar) inserted through a right jugular vein sheath. The micromanometer catheters were calibrated to atmospheric pressure immediately prior to inserting them into the pig.

A 5 French bipolar packing catheter (Daig, Inc., Minnetonka, Minnesota), used to induce ventricular fibrillation (VF) with alternating current at 7 volts and 60 Hz, was inserted through a second right jugular vein sheath and positioned using fluoroscopy in the right ventricular apex. For withdrawal of reference blood samples to measure organ blood flow, a 7F catheter was advanced by femoral arterial access to the aortic arch. Body temperature was monitored continuously via a rectal probe (Yellow Springs Instrument Co., Yellow Springs, Ohio). Core temperatures were maintained between 36.5 and 38.5°C using a heating pad. Five minutes prior to induction of VF, 5000 U of sodium heparin was be administered intravenously.

### 2. Experimental

With 6 pigs/group, using the protocol described hereinabove, the coronary perfusion pressures (CPP) (diastolic aortic minus right arterial pressures) can be seen in Figure 1. The combination of vasopressin + epinephrine leads to more rapid and higher coronary perfusion pressures than either agent alone (Figure 1). In a separate series of experiments with higher concentrations of vasopressin (0.4 units/kg) and epinephrine (0.2 mg/kg) in a porcine model of ventricular fibrillation using standard CPR, myocardial and cerebral blood flows were increased by nearly 40% with the combination of these agents when compared with each drug alone (Figure 2).

### Example 2. Combination Vasopressin/Nitroglycerin Therapy

Applicants have studied the effects of the combination of vasopressin therapy and nitroglycerin therapy in a porcine model of ventricular fibrillation using standard CPR. In this protocol, in which the materials and methods were as described in Example 1, there were 7 pigs/group and an automated device was used to deliver standard CPR. Lindner et al., Circulation, 88, 1254 (1993). Radiolabeled microspheres were used to measure myocardial blood flows during ventricular fibrillation prior to drug therapy and then 90 seconds after drug administration. After 3 minutes of ventricular fibrillation and 2 minutes of standard CPR, regional myocardial blood flow was 18.5 ml/min/100 g in the epicardium and 11 ml/min/100 g in the endocardium. After 10 minutes of ventricular fibrillation, and 7 minutes of standard CPR, vasopressin (0.4 units/kg)± nitroglycerin (5 ug/kg) were administered and blood flow was again measured. Although no differences were seen in total myocardial blood flow, there was a significant increase in endocardial blood flow when nitroglycerin was used. It is the endocardial blood flow which is most comprised during CPR and which benefits from this combination of drugs.

### Example 3. Combination Epinephrine/Zatebradine Therapy

### 1. Summary

This study was designed to assess how a combination of epinephrine and either zatebradine, esmolol, or placebo given during CPR effects hemodynamic variables and myocardial perfusion during the postresuscitation in pigs. Zatebradine administration resulted in a significant reduction in both heart rate and the number of premature ventricular contractions, and improved myocardial contractility. In contrast, esmolol had a significant negative inotropic effect even at a dose that only lead to a moderate heart rate reduction.

### 2. Materials and Methods

### a. Surgical Preparation and Measurements

This study was carried out in twenty-one 12 to 14 week old male domestic pigs, weighing between 23 and 30 kg. The animals were fasted overnight with free access to water. After premedication with azaperone (4 mg/kg IM) and atropine (0.1 mg/kg IM) 30 minutes before surgery, anesthesia was induced with sodium pentobarbital (15 mg/kg) injected into an ear vein. The pigs were then fixed in the dorsal recumbent position, and their tracheas were intubated during spontaneous respiration.

Volume-controlled ventilation (Servo 900, Siemens, Erlangen, Germany) was performed with 65% nitrous oxide in oxygen at 20 breaths/minute with a tidal volume adjusted to maintain normocapnia. Analgesia was achieved with a bolus dose of buprenorphine (0.015 mg/kg) and anesthesia was maintained with a continuous infusion of pentobarbital (0.4 mg/kg/h). Muscle paralysis was achieved by an injection of 10 mg alcuronium after intubation and subsequently as needed. A standard ECG signal was used to record heart rate and the number of premature ventricular contractions. Multiple catheters were used for hemodynamic monitoring.

Cardiac output, right ventricular end-diastolic volume, stroke volume, and right ventricular ejection fraction were determined by the thermodilution technique (5 mL of iced saline into the right atrium) prearrest and at 5, 15, 30, 60 and 180 minutes after ROSC. A micronanometer-tipped catheter (Micro-Tip Pc-350, Millar Houston, TX) was placed into the left ventricle through the left carotid artery for measurement of intraventricular pressure. Left ventricular systolic and end-diastolic pressure, the rate of left ventricular pressure development (dP/dt), and the negative deflection of this curve as a measure of left ventricular relaxation (-dP/dt) were evaluated prearrest and at 5, 15, 30, 60 and 180 minutes after ROSC. In addition, the ratio of (dP/dt) to the left ventricular systolic pressure has been shown to be independent of preload and afterload, and therefore their values have also been calculated. Nejad et al., Cardiovasc. Res., 5, 15 (1971).

All saline-filled catheters were flushed with normal saline containing 5 U heparin/mL at a rate of 3 mL/h (Intraflo II, Abbott Laboratories, North Chicago, II). Using radiolabeled microspheres, myocardial blood flow was measured prearrest and at 5, 15, 30 and 180 minutes after ROSC as previously described by Heyman et al. in Progr. Cardiovasc. Dis., 20, 55(1977). Microspheres (New England Nuclear, Dreieich, Germany, mean diameter 15±1.5µm) were labeled with ¹⁴¹Cereum, ⁹⁶Niobium, ¹⁰³Ruthenium, ⁴⁶Scandium and ⁸⁵Strontium. Each microsphere vial was placed into a water bath and subjected to ultrasonic vibration for 3 minutes before injection. Approximately 5+10⁵ microspheres diluted in 10 mL saline were then immediately injected into the left ventricle. Lidner et al., Am. J. Emerg. Med., 9, 27 (1991). After completion of surgery and before induction of cardiac arrest, 5000 U heparin were administered intravenously to prevent intracardiac clot formation.

### 3. Experimental Protocol

Fifteen minutes before cardiac arrest, the pentobarbital infusion was stopped, the FiO₂ was increased to 1.0, and 0.3 mg buprenorphine was given intravenously. Before induction of cardiocirculatory arrest, hemodynamic parameters, and myocardial perfusion were measured, Ventricular fibrillation was induced by a 50 Hz, 60-V alternating current administered to the thorax via two subcutaneous needle electrodes. Ventilation was stopped at that point.

After 4 minutes of untreated cardiac arrest, manual closed-chest CPR was performed, and mechanical ventilation was resumed with 100% oxygen using identical ventilation parameters as before cardiac arrest. Chest compression rate was 80 per minute. After 3 minutes of CPR, animals were randomly assigned to receive a combination of 45 µg/kg epinephrine and either (a) 0.5 mg/kg zatebradine (n=7), (b) 1 mg/kg esmolol (n=7) or © placebo (n=7). Each drug was given separately through the central venous catheter over a period of 5 seconds, and all investigators were blinded to the drugs. The dosage of 0.5 mg/kg zatebradine was chosen according to study protocols in experimental animals. See, for example, Riley et al., Anesthesiology, 67, 707 (1987). The dosage of 1 mg/kg esmolol was chosen according to the recommendations for the perioperative treatment in patients. Opie et al., "Beta-blocking agents", in Drugs for the Heart, Opie LH(ed), Saunders, Philadelphia, PA. pp 1-30 (1995).

After five minutes of CPR (i.e., 2 minutes after drug administration), three countershocks were initially administered at an energy setting of 3, 4, 6 J/kg, respectively to restore spontaneous circulation. When ventricular fibrillation persisted, epinephrine was administered at the same dose as initially given, and CPR was resumed for another 90 seconds. Subsequently, 3 countershocks at an energy setting of 6 J/kg were delivered. ROSC was defined as coordinated electrical activity and a mean arterial pressure > 50 mm Hg for at least 5 minutes. The Kruskal-Wallis ANOVA was used to determine differences between the 3 groups. Statistical significance was considered to be at the P>0.05 level.

### 4. Results

Prearrest hemodynamic variables did not differ significantly among the 3 groups. During CPR, after administration of a combination of epinephrine, and either zatebradine, esmolol, or placebo, coronary perfusion pressure was 29±4mm Hg, 26±2, 26±4 mm Hg, respectively.

After CPR, defibrillation resulted in ROSC in all animals. Asystole or pulseless electrical activity did not develop in any animal. There was no significant differences in the number of countershocks/animal until ROSC, and the total number of countershocks/animal between groups was observed. During the first 5 minutes of ROSC, the total number of premature ventricular contractions in the zatebradine group (66±30) was significantly lower in comparison with the esmolol group (309±81)(P<0.05) and the placebo group (P<0.05)(251±61).

### a. Hemodynamic variables

As shown in Table 1, hereinbelow, zatebradine caused a significant reduction in heart rate during the entire postresuscitation phase in comparison with the esmolol group and the placebo group. At 5 minutes after ROSC, esmolol administration caused a significant reduction in cardiac output in comparison with the zatebradine group (P<0.05), and the placebo group (P<0.01), and a significant increase in right atrial pressure in comparison with the zatebradine group (P<0.05), and the placebo group (P<0.05). At 5 minutes after ROSC, maximal rate of left ventricular pressure development (dP/dt) and negative deflection rate of Dp/dt (-dP/dt) in the zatebradine group were significantly higher (P<0.05) in comparison with the esmolol group and dP/dt was significantly lower (P<0.05) in comparison with the placebo group. In addition, at 5 minutes after ROSC, the ratio dP/dt to left ventricular systolic pressure in the placebo group was significantly higher in comparison with the zatebradine group (P<0.05), and the esmolol group (P<0.001); and the ratio dP/dt to left ventricular systolic pressure in the zatebradine group was significantly higher (P<0.01) in comparison with the esmolol group. All other hemodynamic variables did not differ significantly among the 3 groups during the period of observation (Table 1).

At 5, 15 and 60 minutes after ROSC in the zatebradine group, stroke volume was significantly higher in comparison with the placebo group (P<0.01 at 5 and 60 minutes after ROSC, P<0.001 at 15 minutes after ROSC). At 5 and 15 minutes after ROSC in the zatebradine group, stroke volume was significantly higher in comparison with the esmolol group (P<0.001). At 5, 15 and 30 minutes after ROSC, right ventricular end-diastolic volume in the placebo group was significantly higher (P<0.05) in comparison with the esmolol group and at 5 minutes after ROSC, right ventricular end diastolic volume in the placebo group was significantly higher (P<0.05) in comparison with the zatebradine group. At 5 minutes after ROSC, right ventricular ejection fraction in the zatebradine group was significantly higher in comparison with the esmolol and placebo group (P<0.001); and at 15, 30 and 60 minutes after ROSC, right ventricular ejection fraction in the zatebradine group was significantly higher in comparison with the placebo group (P<0.05 at 15 and 30 minutes after ROSC, P<0.01 at 60 minutes after ROSC). At 30 and 60 minutes after ROSC, right ventricular ejection fraction in the esmolol group was significantly higher in comparison with the placebo group (P<0.05 at 30 minutes, P<0.01 at 60 minutes after ROSC) (Table 1).

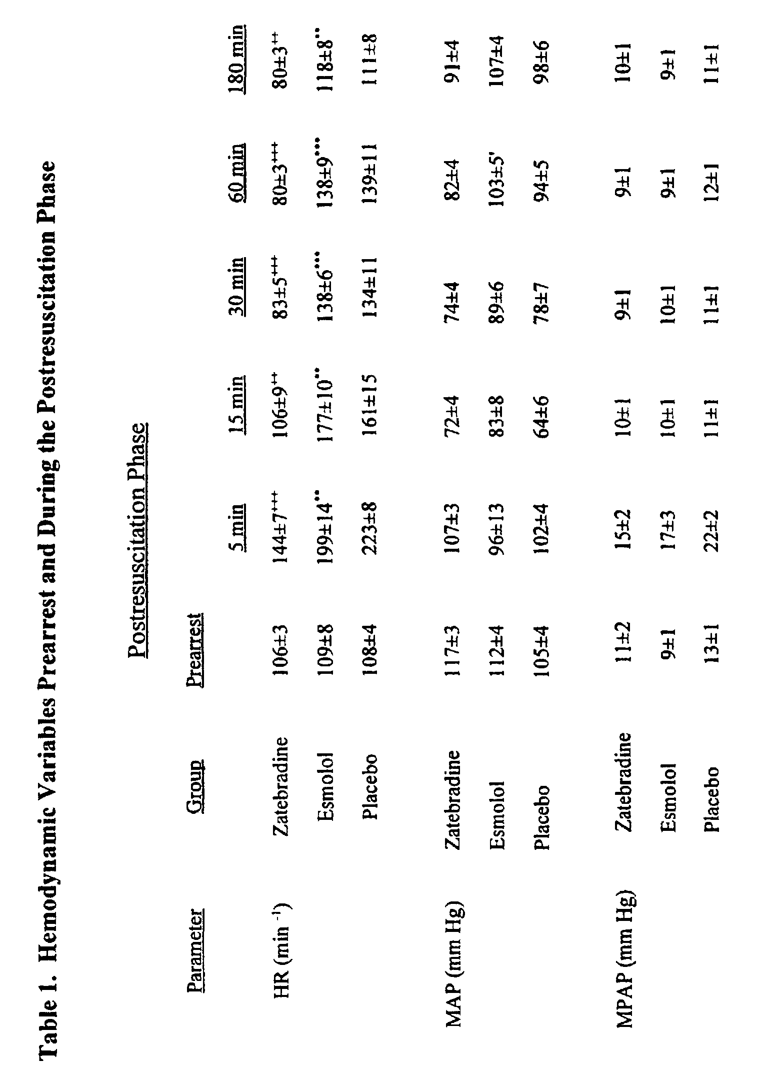

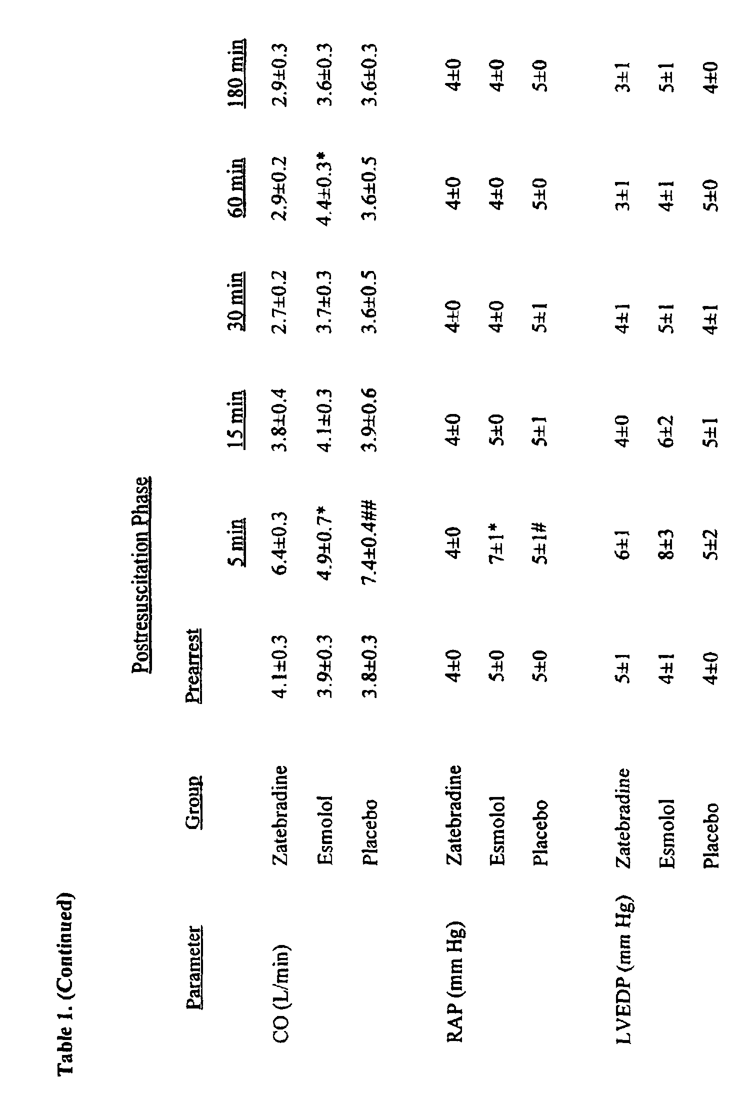

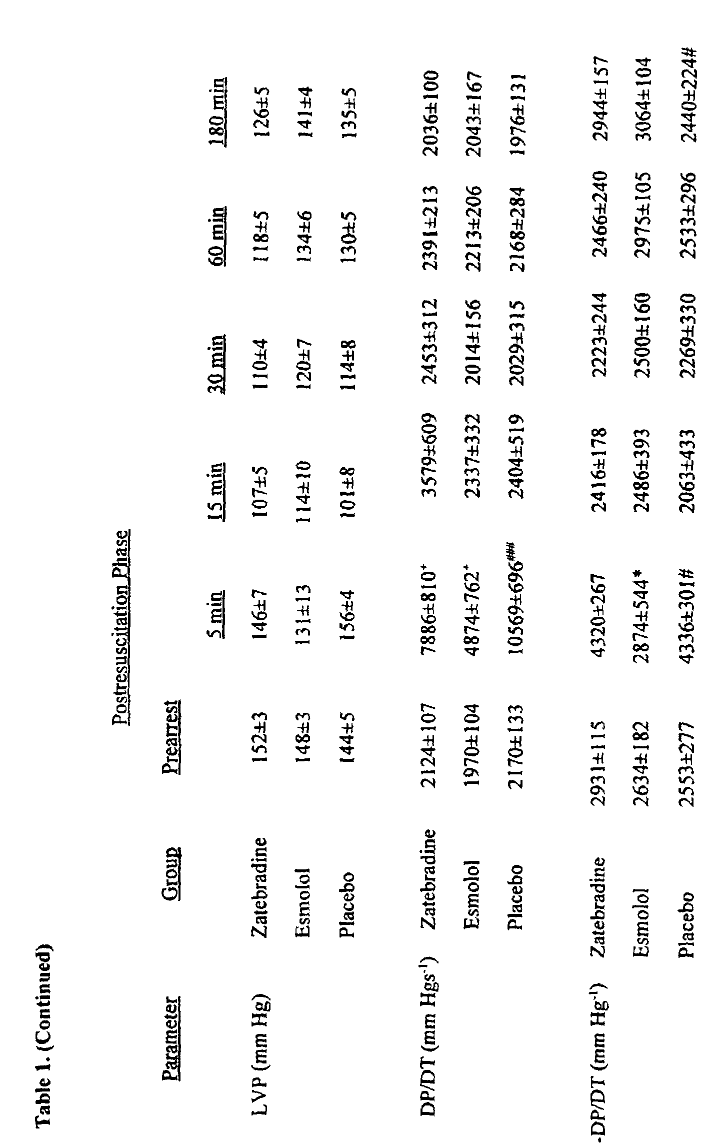

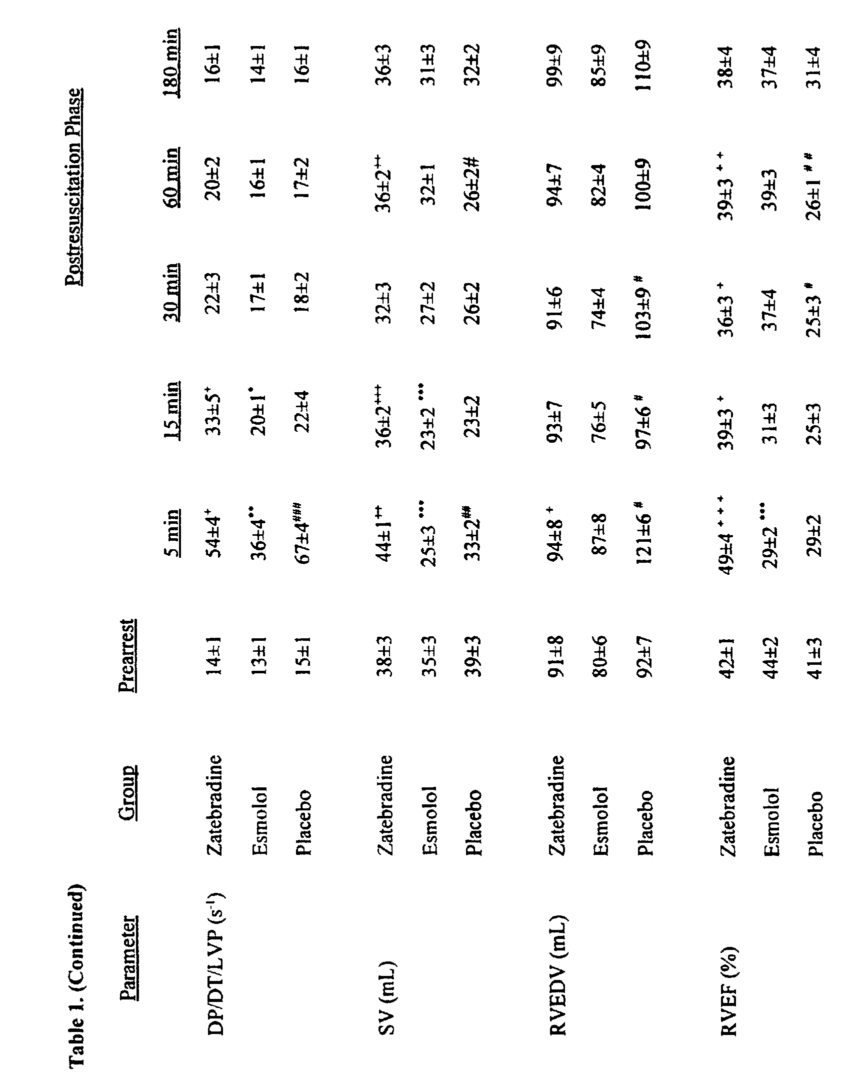

### Table 1 - Legend

Data are mean ± SEM. HR = heart rate; MAP = mean arterial pressure; MPAP = mean pulmonary arterial pressure; CO = cardiac output; RAP = right atrial pressure; LVEDP = left ventricular end-diastolic pressure; LVP = left ventricular systolic pressure; DP/DT = maximal rate of left ventricular pressure development; -DP/DT = negative deflection of DPDT; DP/DT/LVP = ratio of left ventricular pressure development to simultaneous left ventricular pressure; SV = stroke volume; RVEDV = right ventricular end-diastolic volume; RVEF = right ventricular ejection fraction; *P<0.05 esmolol vs zatebradine group; **P<0.01 esmolol vs zatebradine group; ***P<0.001 esmolol vs zatebradine group; +P<0.05 zatebradine vs placebo group; ++P<0.01 zatebradine vs placebo group; +++P<0.001 zatebradine vs placebo group; #P<0.05 placebo vs esmolol group; ##P<0.01 placebo vs esmolol group; ###P<0.001 placebo vs esmolol group at the same point of observation.

### b. Myocardial blood flow

Baseline myocardial blood flow values did not differ significantly among groups. During the postresuscitation phase, there were no significant differences in left ventricular myocardial blood flow between the zatebradine and the placebo group. In contrast, left ventricular myocardial blood flow (mL/g/min), epicardial blood flow, and endocardial blood flow in the esmolol group were significantly lower (P<0.05) (5.6±0.8, 6.4±0.7, 4.9±0.8, respectively) in comparison with the placebo group (11.1±2.1, 12.5±2.3, 9.9±1.5, respectively) at 5 minutes after ROSC. At the same point of observation, the endocardial/epicardial perfusion ratio of the zatebradine group (1.0±0.06)was significantly higher in comparison with the esmolol (0.74±0.06) (P<0.05) and the placebo (0.83±0.05)(P0.05) group. In addition, at 5 minutes after ROSC, the endocardial blood flow/heart rate ratio of the zatebradine and placebo groups were significantly higher than in the esmolol group (P<0.01 zatebradine vs esmolol; P<0.05 placebo vs esmolol). At 15 minutes after ROSC, the endocardial blood flow/heart rate ratio in the zatebradine group was significantly higher (5.4±0.6ml/100g/min/beat) in comparison with the esmolol group (2.3±0.4)(P<0.01) and the placebo group (4.3±0.6(P<0.01).

While only certain preferred embodiments of this invention have been shown and described by way of illustration, many modifications will occur to those skilled in the art and it is, therefore, desired that it be understood that this is intended herein to cover all such modifications that fall within the scope of this invention.

## Claims

1. The use of vasopressin to make a medicament to treat cardiac arrest in conjunction with one or more adrenergic agents, when administered to a patient undergoing cardiopulmonary resuscitation.

2. The use of claim 1 wherein the adrenergic agent is an adrenergic agonist.

3. The use of claim 2, wherein the adrenergic agonist is selected from the group consisting of epinephrine, dopamine, norepinephrine, isoproterenol, phenylephrine, dobutamine and methoxamine, or a combination thereof.

4. The use of claim 3, wherein the adrenergic agonist is epinephrine.

5. The use of claim 1 wherein the adrenergic agent is a PDE inhibitor.

6. The use of claim 5 wherein the PDE inhibitor is aminophylline.

7. The use of claim 1, said use in further conjunction with a bradycardic agent.

8. The use of claim 7 wherein the bradycardic agent is zatebradine, tedisamil, falipamil, mixidine alinidine or a β-adrenergic receptor blocker.

9. The use of claim 8 wherein the bradycardic agent is zatebradine.

10. The use of claim 1 or 7, said use in further conjunction with an organic nitrate.

11. The use of claim 10 wherein the organic nitrate is nitroglycerin.

12. The use of claims 1, 7 or 10, said use in further conjunction with mannitol.

13. The use of claims 1, 7, 10 or 12, further comprising a calcium channel blocker present in an amount effective to inhibit calcium overload.

14. The use of claim 13, wherein the calcium channel blocker is diltiazem, verapamil, or nifedipine.

15. A kit for the treatment of cardiac arrest comprising packaging material containing a first amount of vasopressin, and a second amount one or more adrenergic agents, said amounts each being effective to increase arterial blood pressure so as to enhance blood flow to the brain and heart; and instruction means indicating that said vasopressin and adrenergic agents are to be administered to a patient undergoing cardiopulmonary resuscitation.

16. The kit of claim 15 wherein said first and second amounts are packaged in combination.

17. The kit of claim 15 wherein said first and second amounts are packaged separately.

18. The kit of claim 15, wherein said first amount of vasopressin comprises a unit dosage form of about 10 units to about 120 units of vasopressin.

19. The kit of claim 15 wherein the adrenergic agent is an adrenergic agonist.

20. The kit of claim 19, wherein the adrenergic agonist is selected from the group consisting of epinephrine, dopamine, norepinephrine, isoproterenol, phenylephrine, dobutamine and methoxamine, or a combination thereof.

21. The kit of claim 20, wherein the adrenergic agonist comprises a unit dosage form of about 0.25 mg to 3.0 mg epinephrine.

22. The kit of claim 15 wherein the adrenergic agent is a PDE inhibitor.

23. The kit of claim 22 wherein the PDE inhibitor is aminophylline.

24. The kit of claim 15 further comprising a third amount of a bradycardic agent effective to decrease heart rate.

25. The kit of claim 24 wherein said first, second and third amounts are packaged in combination.

26. The kit of claim 24 wherein said first, second and third amounts are packaged separately.

27. The kit of claim 24 wherein the bradycardic agent is zatebradine, tedisamil, falipamil, mixidine, alinidine or a β-adrenergic receptor blocker.

28. The kit of claim 27 wherein the bradycardic agent is zatebradine and is present at a dose of about 5 mg to about 150 mg.

29. The kit of claims 15 or 24, further comprising an organic nitrate with vasodilatory activity present in an amount effective to increase myocardial blood flow.

30. The kit of claim 29 wherein the vasodilator is nitroglycerin.

31. The kit of claim 30, comprising a unit dosage form of about 10 µg to 300 µg nitroglycerin.

32. The kit of claim 15, 24 or 29, further comprising mannitol in an amount effective to inhibit edema when administered to the patient.

33. The kit of claim 32, comprising a unit dosage form of about 5 g to 50 g mannitol.

34. The kit of claims 15, 24, 29 or 32, further comprising a calcium channel blocker present in an amount effective to inhibit calcium overload in said patient.

35. The kit of claim 34, wherein the calcium channel blocker is diltiazem, verapamil or nifedipine

## Patentansprüche

1. Verwendung von Vasopressin zur Herstellung eines Arzneimittels zur Behandlung von Herzstillstand zusammen mit einem oder mehreren adrenergen Mitteln bei einer Verabreichung an einen Patienten, der einer kardiopulmonalen Wiederbelebung unterzogen wird.

2. Verwendung nach Anspruch 1, worin das adrenerge Mittel ein adrenerger Agonist ist.

3. Verwendung nach Anspruch 2, worin der adrenerge Agonist aus der Gruppe ausgewählt ist, die besteht aus Epinephrin, Dopamin, Norepinephrin, Isoproterenol, Phenylephrin, Dobutamin und Methoxamin oder Kombinationen hiervon.

4. Verwendung nach Anspruch 3, worin der adrenerge Agonist Epinephrin ist.

5. Verwendung nach Anspruch 1, worin das adrenerge Mittel ein PDE Inhibitor ist.

6. Verwendung nach Anspruch 5, worin der PDE Inhibitor Aminophyllin ist.

7. Verwendung nach Anspruch 1, worin die Verwendung in Zusammenhang mit einem bradykarden Mittel erfolgt.

8. Verwendung nach Anspruch 7, worin das bradikarde Mittel Zatebradin, Tedisamil, Falipamil, Mixidin, Alinidin oder ein β-adrenerger Rezeptorblocker ist.

9. Verwendung nach Anspruch 8, worin das bradykarde Mittel Zatebradin ist.

10. Verwendung nach einem der Ansprüche 1 oder 7, wobei die Verwendung ferner zusammen mit einem organischen Nitrat erfolgt.

11. Verwendung nach Anspruch 10, worin das organische Nitrat Nitroglycerin ist.

12. Verwendung nach einem der Ansprüche 1, 7 oder 10, wobei die Verwendung ferner zusammen mit Mannit erfolgt.

13. Verwendung nach einem der Ansprüche 1, 7, 10 oder 12, die ferner einen Calciumkanalblocker umfäßt der in einer Menge vorhanden ist, die zur Hemmung einer Calciumüberladung wirksam ist.

14. Verwendung nach Anspruch 13, worin der Calciumkanalblocker Diltiazem, Verapamil oder Nifedipin ist.

15. Kit zur Behandlung eines Herzstillstands, der Verpackungsmaterial, das eine erste Menge an Vasopressin und eine zweite Menge an einem oder mehreren adrenergen Mitteln enthält, wobei die Mengen zur Erhöhung des arteriellen Blutdrucks wirksam sind, um den Blutfluß zum Gehirn und zum Herzen zu fördern, und Anleitungen umfaßt, die angeben, daß das Vasopressin und die adrenergen Mittel einem Patienten verabreicht werden, der einer kardiopulmonalen Wiederbelebung unterzogen wird.

16. Kit nach Anspruch 15, worin die ersten und zweiten Mengen in Kombination verpackt sind.

17. Kit nach Anspruch 15, worin die ersten und zweiten Mengen getrennt verpackt sind.

18. Kit nach Anspruch 15, worin die erste Menge an Vasopressin eine Einheitsdosierungsform von etwa 10 Einheiten bis etwa 120 Einheiten Vasopressin umfaßt.

19. Kit nach Anspruch 15, worin das adrenerge Mittel ein adrenerger Agonist ist.

20. Kit nach Anspruch 19, worin der adrenerge Agonist ausgewählt ist aus der Gruppe, die besteht aus Epinephrin, Dopamin, Norepinephrin, Isoproterenol, Phenylephrin, Dobutarnin und Methoxamin oder einer Kombination hiervon.

21. Kit nach Anspruch 20, worin der adrenerge Agonist eine Einheitsdosierungsform mit etwa 0,25 mg bis 3,0 mg Epinephrin enthält.

22. Kit nach Anspruch 15, worin das adrenerge Mittel ein PDE Inhibitor ist.

23. Kit nach Anspruch 22, worin der PDE Inhibitor Aminophyllin ist.

24. Kit nach Anspruch 15, der ferner eine dritte Menge eines bradykarden Mittels enthält, die zur Verringerung der Herzfrequenz wirksam ist.

25. Kit nach Anspruch 24, worin die ersten, zweiten und dritten Mengen in Kombination verpackt sind.

26. Kit nach Anspruch 24, worin die ersten, zweiten und dritten Mengen getrennt verpackt sind.

27. Kit nach Anspruch 24, worin das bradykarde Mittel Zatebradin, Tedisamil, Falipamil, Mixidin, Alinidin oder ein β-adrenerger Rezeptorblocker ist.

28. Kit nach Anspruch 27, worin das bradykarde Mittel Zatebradin ist und in einer Dosis von etwa 5 mg bis etwa 150 mg vorhanden ist.

29. Kit nach einem der Ansprüche 15 oder 24, der ferner ein organisches Nitrat mit vasodilatatorischer Aktivität enthält, das in einer Menge vorhanden ist, die zur Erhöhung des myokardialen Blutflusses wirksam ist

30. Kit nach Anspruch 29, worin der Vasodilatator Nitroglycerin ist.

31. Kit nach Anspruch 30, der eine Einheitsdosierungsform mit etwa 10 µg bis 300 µg Nitroglycerin enthält.

32. Kit nach einem der Ansprüche 15, 24 oder 29, der ferner Mannit in einer wirksamen Menge umfaßt, um Ödeme bei einer Verabreichung an einen Patienten zu verhindern.

33. Kit nach Anspruch 32, der eine Einheitsdosierungsform von etwa 5 g bis 50 g enthält.

34. Kit nach einem der Ansprüche 15, 24, 29 oder 32, der ferner einen Calciumkanalblocker in einer Menge enthält, die zur Hemmung der Calciumüberladung bei diesem Patienten wirksam ist.

35. Kit nach Anspruch 34, worin der Calciumkanalblocker Diltiazem , Verapamil oder Nifedipin ist.

## Revendications

1. Utilisation de la vasopressine pour fabriquer un médicament destiné à traiter les arrêts cardiaques conjointement avec un ou plusieurs agents adrénergiques, lorsqu'il est administré à un patient en réanimation cardiopulmonaire.

2. Utilisation selon la revendication 1, dans laquelle l'agent adrénergique est un agoniste adrénergique.

3. Utilisation selon la revendication 2, dans laquelle l'agoniste adrénergique est choisi dans le groupe comprenant l'épinéphrine, la dopamine, la norépinéphrine, l'isoprotérénol, la phényléphrine, la dobutamine et la méthoxamine, ou une combinaison de celles-ci.

4. Utilisation selon la revendication 3, dans laquelle l'agoniste adrénergique est l'épinéphrine.

5. Utilisation selon la revendication 1, dans laquelle l'agent adrénergique est un inhibiteur de la PDE.

6. Utilisation selon la revendication 5, dans laquelle l'inhibiteur de la PDE est l'aminophylline.

7. Utilisation selon la revendication 1, dans laquelle ladite utilisation s'effectue en outre conjointement avec un agent bradycardique.

8. Utilisation selon la revendication 7, dans laquelle l'agent bradycardique est la zatébradine, le tédisamil, le falipamil, la mixidine, l'alinidine ou un inhibiteur du récepteur β-adrénergique.

9. Utilisation selon la revendication 8, dans laquelle l'agent bradycardique est la zatébradine.

10. Utilisation selon la revendication 1 ou 7, ladite utilisation s'effectuant conjointement avec un nitrate organique.

11. Utilisation selon la revendication 10, dans laquelle le nitrate organique est la nitroglycérine.

12. Utilisation selon les revendications 1, 7 ou 10, ladite utilisation s'effectuant en outre conjointement avec du mannitol.

13. Utilisation selon les revendications 7, 10 ou 12, comprenant en outre un antagoniste calcique présent dans une quantité efficace pour inhiber la surcharge en calcium.

14. Utilisation selon la revendication 13, dans laquelle l'antagoniste calcique est le diltiazem, le vérapamil ou la nifédipine.

15. Trousse pour le traitement des arrêts cardiaques comprenant un conditionnement contenant une première quantité de vasopressine et une deuxième quantité d'un ou de plusieurs agents adrénergiques, lesdites quantités étant chacune efficace pour augmenter la pression sanguine artérielle afin d'améliorer la circulation sanguine vers le cerveau et le coeur ; et des moyens indicateurs indiquant que ladite vasopressine et lesdits agents adrénergiques doivent être administrés à un patient en réanimation cardiopulmonaire.

16. Trousse selon la revendication 15, dans laquelle lesdites première et deuxième quantités sont conditionnées de manière combinée ;

17. Trousse selon la revendication 15, dans laquelle lesdites première et deuxième quantités sont conditionnées séparément.

18. Trousse selon la revendication 15, dans laquelle ladite première quantité de vasopressine comprend une forme galénique unitaire d'environ 10 unités à environ 120 unités de vasopressine.

19. Trousse selon la revendication 15, dans laquelle l'agent adrénergique est un agoniste adrénergique.

20. Trousse selon la revendication 19, dans laquelle l'agoniste adrénergique est choisi dans le groupe comprenant l'épinéphrine, la dopamine, la norépinéphrine, l'isoprotérénol, la phényléphrine, la dobutamine et la méthoxamine ou une combinaison de ceux-ci.

21. Trousse selon la revendication 20, dans laquelle l'agoniste adrénergique comprend une forme galénique unitaire d'environ 0,25 mg à 3,0 mg d'épinéphrine.

22. Trousse selon la revendication 15, dans laquelle l'agent adrénergique est un inhibiteur de la PDE.

23. Trousse selon la revendication 22, dans laquelle l'inhibiteur de la PDE est l'aminophylline.

24. Trousse selon la revendication 15, comprenant en outre une troisième quantité d'un agent bradycardique efficace pour ralentir la fréquence cardiaque.

25. Trousse selon la revendication 24, dans laquelle lesdites première, deuxième et troisième quantités sont conditionnées de manière combinée.

26. Trousse selon la revendication 24, dans laquelle lesdites première, deuxième et troisième quantités sont conditionnées séparément.

27. Trousse selon la revendication 24, dans laquelle l'agent bradycardique est la zatébradine, le tédisamil, le falipamil, la mixidine, l'alinidine ou un inhibiteur du récepteur β-adrénergique.

28. Trousse selon la revendication 27, dans laquelle l'agent bradycardique est la zatébradine et est présent selon une dose d'environ 5 mg à environ 150 mg.

29. Trousse selon la revendication 15 ou 24, comprenant en outre un nitrate organique ayant une activité vasodilatatrice présent dans une quantité efficace pour augmenter la circulation sanguine myocardique.

30. Trousse selon la revendication 29, dans laquelle le vasodilatateur est la nitroglycérine.

31. Trousse selon la revendication 30, comprenant une forme galénique unitaire d'environ 10 µg à 300 µg de nitroglycérine.

32. Trousse selon la revendication 15, 24 ou 29, comprenant en outre du mannitol dans une quantité efficace pour inhiber l'oedème lorsqu'il est administré au patient.

33. Trousse selon la revendication 32, comprenant une forme galénique unitaire d'environ 5 g à 50 g de mannitol.

34. Trousse selon la revendication 15, 24, 29 ou 32, comprenant en outre un antagoniste calcique présent dans une quantité efficace pour inhiber une surcharge de calcium chez ledit patient.

35. Trousse selon la revendication 34, dans laquelle l'antagoniste calcique est le diltiazem, le vérapamil ou la nifédipine.
